# EUROPEAN PATENT APPLICATION

(11) **EP 4 667 022 A1**
(43) Date of publication of application: **24.12.2025**
(21) Application number: 25180386.2
(22) Date of filing: 03.06.2025
(51) Int. Cl.: A61L 24/10, A61L 26/00

(54) **METHOD OF MANUFACTURING AN AQUEOUS SOLUTION OF BOVINE SERUM ALBUMIN, ADHESIVE COMPOSITION CONTAINING AN AQUEOUS SOLUTION OF BOVINE SERUM ALBUMIN PREPARED THEREBY, AND USE OF THE AQUEOUS SOLUTION OF BOVINE SERUM ALBUMIN TO PRODUCE TISSUE ADHESIVE**

(30) Priority: 17.06.2024 PL 44886624
(71) Applicant: Grena Biomed Limited, Nottinghamshire NG7 7HP (GB)
(72) Inventor: Matak, Damian, 96-313 Budy Zosine (PL)
(74) Representative: Kondrat, Mariusz

(57) **Abstract**

The object of the invention is a method of manufacturing an aqueous bovine albumin solution of 30-60% w/v, where bovine albumin powder is mixed with the solution as follows: a. the aqueous solution and bovine albumin powder in such quantities as to provide a concentration of 30-60% w/v are introduced into a sterile plastic bag, b. the bag is closed tightly so that air is removed from the inside of the bag, c. optionally, the powder is initially manually distributed in the hands with water so that the powder is distributed throughout the volume of the aqueous solution, d. manual removal of all lumps of bovine albumin powder in the aqueous solution is carried out for 2-3h by crushing the lumps between the surfaces of the bag. It is also an object of the invention to provide an adhesive composition comprising an aqueous solution of bovine albumin prepared in this manner and an aqueous solution of a di- or multifunctional aldehyde. It is also an object of the invention to use an aqueous solution of bovine albumin prepared by the above method to obtain a tissue adhesive.

## Description

The object of the invention is a method for obtaining an aqueous solution of bovine albumin for use in an adhesive composition, in particular a tissue adhesive useful in medical practice and human or animal surgery.

Various types of tissue adhesives are known in the prior art. The most common are three types of adhesives namely cyanoacrylates, protein-aldehyde compositions and fibrin-based adhesives. Such adhesives are used as an adjunct to standard tissue repair methods such as sutures, staples, electrocautery and patching to seal, bond and reinforce tissue. They can be used alone to attach surgical mesh, reinforce, repair or seal damaged parenchyma as a substitute for ineffective or impractical techniques: vascular surgery, cardiac surgery, thoracic surgery, general surgery, genitourinary surgery, neurosurgery, ENT surgery, spinal surgery.

Tissue adhesives based on bovine albumin are available on the market. The combination of purified bovine albumin and aldehyde in the optimum ratio and the precise mixing of the ingredients inside the applicator produces a completely biocompatible, flexible surgical adhesive. An adhesive of this type is also described in patent EP0650512B1. The adhesive composition according to this disclosure contains a plasma protein or globular protein and a bifunctional or multifunctional aldehyde, the composition not containing chlorohexidine digluconate and not containing insulin. Advantageously, the protein comprises albumin. The aldehyde component means a dialdehyde selected from the group consisting of glyoxal, succinic aldehyde, glutaraldehyde, malealdehyde and phthalaldehyde. An applicator for delivering an adhesive composition to a tissue surface comprises a plasma protein or globular protein and a bifunctional or multifunctional aldehyde placed therein, the applicator comprising first and second separate chambers, the protein being placed in the first chamber and the aldehyde being placed in the second chamber of the applicator. The protein and aldehyde are mixed together to form a composition before it is applied to the tissue surface. Bond formation is rapid and usually takes less than one minute. The adhesion formed is strong and generally provides bonds with a tear strength of about 400 g/cm² to about 600 g/cm².

The adhesive therefore has two components: a first component containing albumin and a second component containing aldehyde. As the albumin in the adhesive, bovine serum albumin, BSA (BSA - from Bovine Serum Albumin), is used according to the state of the art. To produce the first component of the adhesive, bovine serum albumin is dissolved in an inert carrier, for example water, a dilute buffer and/or a salt solution. This solution has an albumin concentration of, for example 35-45% w/v. As a second component, glutaraldehyde, for example, is used in practice in the form of a solution with a concentration of 5-15% w/v. The problem in obtaining such adhesives is to produce an albumin solution with a concentration equal to or higher than 30% w/v.

When bovine albumin is mixed with aqueous solutions to obtain an albumin concentration equal to or greater than 30 % w/v, foam is formed. The problem of protein denaturation in foam has long been observed [Journal of Colloid and Interface Science 215, 323-332 (1999), J. R. Clarkson, Z. F. Cui,1 and R. C. Darton, Protein Denaturation in Foam]. Protein denaturation was found to be mainly due to surface denaturation occurring at the gas-liquid interface and the degree of denaturation correlates directly with interfacial exposure.

The inventors of the present invention have confirmed observations known to the state of the art that when beef albumin is mixed with an aqueous solution according to recipes known to the state of the art, foaming of the solution occurs [Food Hydrocolloids 21 (2007) 495-506, L.A. Glaser, A.T. Paulson, R.A. Speers, R.Y. Yadab, D. Rousseau, Foaming behaviour of mixed bovine serum albumin-protamine systems]. During foam formation, proteins (i) diffuse into the air-water interface, congeal and reduce surface tension; (ii) partially unfold and change orientation so that polar groups are oriented towards the polar phase; and (iii) interact with each other through non-covalent interactions and potentially covalent bonds to form a continuous viscoelastic layer. Protein molecules undergo denaturation, conformational changes and also interactions through the formation of covalent and non-covalent bonds, resulting in altered oligomeric structures. Meanwhile, the amount of these adverse processes occurring in albumin during its mixing with water translates into the quality of the tissue adhesive. It has been established that the presence of an adequate amount of albumin monomers is crucial for obtaining the desired adhesive parameters: adhesion and cohesion strengths, and polymerisation rate after mixing with aldehyde.

Until now, in practice, the dissolution of albumin in water for subsequent use in tissue adhesives has most often been carried out slowly, by gradually adding albumin in powder form to the solution and stirring gently - so that as little foam as possible is formed. This is a very time-consuming process, even taking several days. Only then can denaturation and/or polymerisation of the protein be minimised. However, prolonged stirring causes the solution to be exposed to ambient conditions for a long time which in turn causes microorganisms to grow in the solution. In addition, the equipment needs to be washed after each mixing process of several days, which is quite difficult with such a high concentration of albumin. High concentrations of albumen in aqueous solution are very viscous. The residue left behind quickly dries to form a 'crust' that is difficult to wash. It is worth mentioning that the equipment for mixing BSA when making tissue glue must be highly clean. Tissue glue is a high-risk medical device, implanted inside the body. There is no way to manufacture glue under conditions of unclean residues or dirt.

Various methods are proposed in the prior art obtaining such solutions with a high concentration of albumin. For the purpose of the present application, high concentration of bovine albumin in aqueous solutions is understood to be a concentration equal to or higher than 30% w/v. The preparation of concentrations of a few to several per cent is not technologically problematic.

Dissolution of bovine albumin can be carried out by various methods. The standard ones described in the literature involve slowly adding bovine albumin powder to the liquid and stirring gently to avoid foaming. This is usually done in a suitable vessel dedicated to the scale [https://www.labmanager.com/7-tips-for-suspending-bsa-in-solution-7199,
https://www.aatbio.com/resources/faq-frequently-asked-questions/how-do-i-dissolve-bsa-powder].

Such a process to achieve a high protein concentration (=> 30% w/v) is inefficient. The reason for this is the long time of the dissolution process, and the associated risk of microbial growth - longer time and room temperature are ideal conditions for their development.

To mix bovine albumin with water, according to the state of the art, slow and gradual addition of powder to the solution and periodic gentle agitation of the container is used. The state of the art also describes a method for dissolving protein by layering (colloquially, sandwiching). Such a method involves pouring a suitable amount of aqueous solution into a bottle, adding a layer of powder, over which another portion of water is spread, and then another layer of powder, and so on. In practice, this is an ineffective method for achieving higher concentrations, because lumps of BSA form that are very difficult to break up (they have the consistency of hard candy). In this method, it is not possible to evenly disperse the water on the surface of the powder and vice versa. This method is also time-consuming.

One bovine albumin supplier suggests making the solution to specification by gradually and slowly adding the powder, however, at such a high concentration of protein the use of any stirrer will cause foaming. It is also suggested to increase the temperature of the solution, which is risky due to the acceleration of microbial growth rates in the solution and denaturation of the protein. [https://phb1.com/content/uploads/2023/05/Dissolving-BSA-Into-Solution-White-Paper.pdf]

In view of these problems observed in known methods of producing bovine albumin solution in aqueous solution at or above 30% w/v, there is a need for a simple, relatively rapid process of mixing these components, using simple instrumentation, while ensuring conditions that do not lead to denaturation, protein polymerisation or microbial contamination of the solution.

Thus, it is an object of the invention to provide a method for producing an aqueous solution of bovine albumin with a concentration of more than 30% w/v so that the process is simple, relatively fast, using simple instrumentation, while ensuring conditions that do not lead to protein denaturation, polymerization as well as infestation of the solution with microorganisms. A further aim of the invention is to provide an adhesive composition containing bovine albumin solution obtained by such a method and to use this solution for the preparation of a tissue adhesive. Such an adhesive has improved properties as demonstrated in the Examples.

The object of the invention is a method for producing an aqueous solution of bovine albumin of 30-60% w/v, where the bovine albumin powder is mixed with the aqueous solution, characterised in that the mixing is carried out as follows:
a. an aqueous solution and bovine albumin powder in such quantities as to ensure a concentration of 30-60 % w/v are introduced into a sterile plastic bag,
b. the bag is closed tightly to remove the air inside the bag,
c. optionally, the powder is initially spread manually in the hands with water, so that the powder is distributed throughout the volume of the water solution,
d. manual removal of all lumps of bovine serum albumin powder in aqueous solution is carried out for 2-3h by crushing the lumps between the surfaces of the bag.

Advantageously, the manual removal of all lumps of bovine albumin powder in aqueous solution is carried out with the hands by crushing these lumps with the fingers between the surfaces of the bag.

Also, the manual removal of all lumps of bovine albumin powder in aqueous solution is advantageously carried out by crushing the lumps by rolling with a roller.

The aqueous solution is preferably water, diluted buffer and/or salt solution.

Advantageously, the aqueous bovine albumin solution obtained is a 45% w/v solution.

The object of the invention is also an adhesive composition comprising an aqueous solution of bovine albumin prepared by the above method and an aqueous solution of a di- or multifunctional aldehyde.

Advantageously, the aqueous solution of bovine albumin in the adhesive composition is an aqueous solution of 45% w/v, the solution of di- or polyfunctional aldehyde is an aqueous solution of 10% w/v of glutaraldehyde and the ratio of bovine albumin and glutaraldehyde solution is 4:1.

It is also an object of the invention to use an aqueous solution of bovine albumin prepared by the above method to obtain a tissue adhesive.

The technical problem that the invention solves is the problem of miscibility of albumin in aqueous solution, especially when obtaining very concentrated solutions (above 30% w/v). The formation of oligomers or the subsequent denaturation of the protein during the various mixing techniques, as well as the infection of the solution with microorganisms, result in a deterioration of the quality of this solution, which translates into the quality of tissue adhesive, of which bovine albumin solution in water is a component. The invention addresses these problems.

The target tissue glue is made by mixing albumin and aldehyde just before use. The tissue adhesive is applied to the tissue to bond it, e.g. after cutting during surgery. Of course, it is better if the adhesive has better adhesive properties, but it is a medical product that must also meet microbiological safety requirements. The invention addresses this problem. The adhesive formed from a composition containing bovine albumin solution made by the inventive method solves the above problems comprehensively.

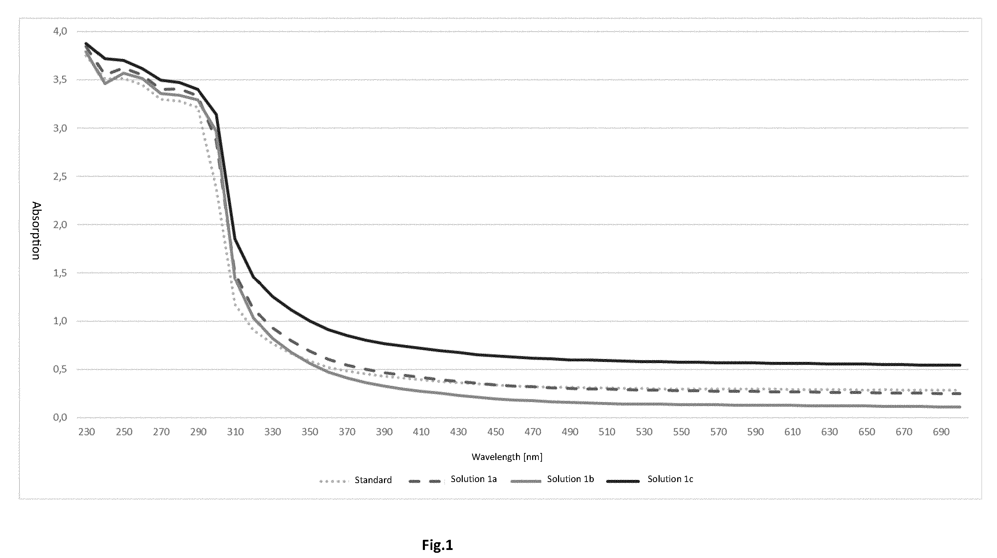

The effect of the invention is shown by means of the test results described in the Examples. Example 6 refers to figure 1.

Fig 1. shows a graph of the wavelength dependence of absorbance for the standard, Solution 1a, Solution 2 and Solution 3 (UV-Vis test result); the light grey dotted line indicates the standard, the dark grey dashed line indicates Solution 1a (invention), the light grey solid line indicates Solution 2 (reference), the black solid line indicates Solution 3 (reference). The graph shows the structural similarity of the components of Solution 1a to the standard.

The bovine albumin can be any bovine albumin available on the market. To substantiate the invention, serum bovine albumin purchased from Bovogen Biologicals Pty Ltd was used.

A di- or multifunctional aldehyde is understood to be an aldehyde containing more than one aldehyde group. However, they can also be other aldehydes that polymerise with bovine albumin, e.g. glyoxal (OHC-CHO), malonoaldehyde CH₂(CHO)₂), succinic aldehyde (OHC(CH2)2CHO), glutaraldehyde (OHC(CH₂)₃CHO), adipoaldehyde (OHC(CH₂)₄CHO), phthaldialdehyde (OHC-C₆H₄-CHO), pyridine-2,3-dialdehyde, thiophene-2,3-dialdehyde, furano-2,5-dialdehyde, terephthalaldehyde (C₆H₄(COH)₂. The most preferable is glutaraldehyde.

The water used to dissolve albumin must, due to the medical use of the solution, have parameters that comply with the European Pharmacopoeia for water for injection, https://rmbio.com/products/sterile-purified-water. Of course, when using the method according to the invention to produce albumin solution in aqueous solutions for other than medical purposes, the water need not be of this quality. The method will be suitable for water of general public quality.

The diluent for bovine albumin can be pure water, a diluted buffer and/or a salt solution such as saline. Possible buffers are, for example, PBS (and. phosphate-buffered saline) with a composition of NaCl 0.137 M, KCI 0.0027 M, Na₂HPO₄0.01 M, KH₂PO₄0.0018 M. The solubility of albumin in high concentrations is not substantially affected by this small addition of buffering compounds and/or salts to water. The method of obtaining a solution of albumin in water translates directly into dilute buffers and/or salt solutions in water.

The aldehyde solution for making the tissue adhesive can be prepared in aqueous solutions, especially water.

The bags in which bovine albumin is mixed with the aqueous solution must be inert to the solution. These may be, for example, bags made of polyethylene or polyethylene blends closed with, for example, a zipper closure or by wrapping the bag. Bags of any other plastic inert to the proteins will be suitable. The closure of the bag is also free, provided that it ensures an airtight seal.

The roller used to knead the albumin suspension in water can be acrylic. Other rollers will, of course, also be suitable for carrying out the method according to the invention. These can be fluted rollers containing hammered spears. In general, the width of the roller should preferably be less than the width of the bag, preferably the width of the roller should be ¾ of the width of the bag. Then, when rolling through the centre of the bag, the solution can flow freely at the edges of the length of the bag. Of course, the roller may be less than ¾ of the width of the bag, e.g. ½ of the width of the bag. When performing the method according to the invention, the roller is not in contact with the solution.

The method according to the invention solved the state-of-the-art problem related to foaming of bovine albumin solution with high concentration mixed by known methods. This minimised the amount of denatured protein and protein oligomers, which were formed in significant amounts when bovine albumin was mixed with water according to the state of the art methods. Making the solutions according to the state of the art immediately showed foam, the amount of which increased with mixing time. The number of steps performed in the methods from the prior art (portions of albumin introduced into the water) was large, up to a few dozen. The mixture was open to external conditions and exposed to micro-organisms from the air, as well as those present on the worker or equipment used (a tool had to be used several/some times to transfer the albumin powder into the water container). After all, when increasing the number of portions of albumin added to the water from a few to several dozen, slightly less foaming was observed, but the amount of bacteria in the solution increased. Each of the known methods tested by the developers did not produce a satisfactory result. The glue obtained from highly concentrated bovine albumin solutions obtained by the known methods was not of good quality. The more foam - i.e. denatured protein and protein polymers as well as bacteria present in the solution - the poorer the adhesive properties. The poorer quality of the adhesive was manifested by deteriorated adhesive properties, which can be explained by the reduced amount of albumin monomers active for polymerisation with aldehyde.

The solution of albumin in water obtained by the method according to the invention can be used for the preparation of tissue adhesives, which require highly concentrated bovine albumin solutions. It is clear that the presence of the smallest possible amount of denatured and structurally modified albumin, is associated with the presence in the solution of the largest possible amount of monomers capable of polymerisation with aldehyde. This translates into improved tissue adhesive properties. An increase of more than 20 % in the maximum load retention was observed for the adhesive sample according to the invention relative to the adhesive sample obtained by the state-of-the-art method. This demonstrates the better adhesive properties of the adhesive obtained from the bovine albumin solution made by the method according to the invention. In addition, the reduction in the number of microorganisms in the aqueous albumin solution clearly translates into increased safety in the use of the adhesive obtained from the bovine albumin solution made according to the inventive method as a medical device.

Of course, such a solution of albumin in water obtained according to the inventive method can also be used in other fields of technology where the quality of this solution is important at high concentrations equal to or higher than 30% w/v of albumin in aqueous solutions.

It is also evident that an aqueous solution of bovine albumin with a concentration of less than 30% w/v can be obtained by the method according to the invention. However, with these low-concentration solutions, no such foaming problems are observed as with highly-concentrated solutions. Bovine albumin at low concentrations dissolves much faster in aqueous solutions and there is also little risk of contamination of such solutions with microorganisms from the external environment.

Benefits of the method of obtaining the solution according to the invention:
- a simple procedure that can be carried out manually, using commonly available equipment/objects, at room temperature,
- shortening the process of obtaining a homogeneous suspension from several days to a few hours (approximately 2h),
- reducing the number of micro-organisms present in solution by a factor of ten,
- keeping foaming to a minimum,
- obtaining a highly concentrated solution of BSA that resembles fresh protein in its native form,
- reduction in the amount of decentralised protein,
- time and cost savings (reduction in time, use of simple objects),
- the possibility of obtaining a batch of product in a maximum of 3h, taking into account additional storage of the solution after mixing in the refrigerator,
- using of disposable, sterile materials (no washing), which ensures reproducible process conditions,
- when carrying out the method according to the invention, only the inside of the sterile bag is in direct contact with the solution and not, as in the case of mixers, the various parts of the device.

The figures used in this description for total manner time should be understood as +/- 15 min.

### EXAM PLES

### Reagents, materials and instrumentation used

The following materials and equipment were used to make the method according to the invention.

Serum bovine albumin was obtained from Bovogen Biologicals Pty Ltd[https://bovogen.com/our-products/purified-proteins/bovine-serum-albumin/]. The form of albumin is a white or amber powder, purity >98%, pH of 10% solution 6.5-7.2.

The water used was injection water purchased from Rocky Mountain Biologicals, LLC [https://rmbio.com/products/sterile-purified-water].

The bag used in the examples is a sterile polyethylene compound bag [https://www.whirl-pak.com/product/whirl-pak-write-on-sterilized-bags/].

The examples use the acrylic roller [https://www.stormplastyczny.pl/pl/p/Akrylowy-walek-do-modeliny-Fimo/5828]. It is also possible to use other rollers. E.g. rollers of the roller/massage roller type with tabs will be suitable. Rollers such as the Gymtek EVA roller, massage roller [Gymtek EVA roller, massage roller - black/white - Sport-Shop.co.uk] are suitable.

Suitable for crushing lumps of BSA in aqueous solution will be any available roller that allows the crushing of loose lumps but will not damage the bag.

Glutaraldehyde sourced from VWR Avantor^{®} [https://us.vwr.com/store/product/22071133/glutaraldehyde-10-in-aqueous-solution-em-grade

PBS phosphate-buffered saline was obtained from [https://pl.vwr.com/store/product/8606883/null].

TSA - Tryptonic Soy Agar [https://www.sigmaaldrich.com/PL/pl/product/sial/22091?utm source=google&utm medium=cpc& utm campaign=8692675846&utm content=92935475248&gclid=CiwKCAiAxaCvBhBaEiwAvsLmWNzE TVxDNIELkGc8p-WVM6uMIuHHoXHJ1hIvUV5UE4PUeJ1TG8vcRoCctoQAvD BwE]

Laemmli Sample Buffer - Laemmli sample buffer sourced from Bio-Rad's [https://www.bio-rad.com/webroot/web/pdf/Isr/literature/4006028_G.pdf].

Mini-Protean Tetra Cell for the SDS-Page method [https://www.bio-rad.com/enpl/product/mini-protean-tetra-cell?ID=d5d6580e-b8f6-4f1c-bbc4-f63d360ea7881

TGS (Tris/glycine/SDS)- 10x pre-mixed electrophoresis buffer, contains 25 mM Tris, 192 mM glycine, 0.1% SDS (sodium dodecyl sulfate denaturing agent), pH 8.3 after 1x dilution with water [https://www.bio-rad.com/en-pl/sku/1610732-10x-tris-glycine-sds?ID=1610732].

Bio-Safe Coomassie G-250 - staining powder in SDS-Page, [https://www.bio-rad.com/ja/sku/1610406-coomassie-brilliant-blue-g-250?ID=1610406&s_kwcid=AL!18120131688732177104!!!g!!&WT.mc_id=240129040766&WT.srch=1 &WT.knsh_id=ee0e71e9-850c-400d-808d-f09cc60blcfo&gad source=1&gclid=CjwKCAiAxaCvBhBaEiwAvsLmWNQ691JZznKucfMkVO-65hPWky2z-KZnhyh23MeOo-kU8HRp7zw3-BoCH-0QAvD_BwE]

BSA template for SDS-Page - https://www.sigmaaldrich.com/PL/pl/product/sigma/p0834?utm_source=google&utm_medium=cpc &utm_campaign=8691857245&utm_content=98396010471&gclid=CjwKCAiAxaCvBhBaEiwAvsLmWJw gW21mqAViuuCgFuBW3EuPLIkzNKvjHKUSKpnRcB3EIvkMBEuyEhoCgg8QAvD_BwE

UV-Vis spectrophotometer uQuant Bio-Tek, Universal Microplate Spectrophotometer with single-channel readout and test system [https://www.artisantg.com/Scientific/71777-1/Bio-TekuQuant-Universal-Microplate-Spectrophotometer].

The stirrers used to obtain the reference solutions are commercially available - a reference to a market product is indicated in the examples.

### Example 1. Solution number 1a, 1b and 1c according to the invention

### Solution 1a

A measured amount of water (1000 ml) was poured into the Whirl-Pak^{®} bag https://www.whirl-pak.com/product/whirl-pak-write-on-sterilized-bags/ (approx. 2 I in volume, bag size 19 cm x 38 cm, sealable by wrapping and crimping with a wire) and then BSA powder (675g) was added to obtain a final solution of 45% w/v. The bag was closed manually using a wrap-around closure, while pressing the mixture into the bag to remove any air above the mixture before closing. The advantage of using a wrap-around bag is that the height of the closure can be adjusted depending on how much water and how much powder is in the bag. The final closure is made to the initial volume of water, added first, and powder, poured into the water.

There will still be air in the bag, if only in the space between the particles of bovine albumin powder. Therefore, it is not as important (nor is it possible) to remove all the air from above the mixture. The initial mixture is a suspension of bovine albumin powder in water, lumps of powder are visible. The presence of these lumps and the poor wettability of bovine albumin powder with water are responsible for the miscibility problem of these agents.

### Manual method using hands

In the initial phase, the BSA powder was gently wetted with water by gently manually repositioning and kneading the contents of the bag in the hands so that the powder was distributed throughout the volume of water. Subsequently, gentle kneading of the bag contents was started with the hands distributing the loose powder in the water. Once the BSA powder was partially 'broken down', each lump of BSA was mashed separately. The grinding was carried out by kneading the lumps with the fingers between the two surfaces of the bag.

For harder lumps, you can use an acrylic roller or another roller from those listed in the description.

The process was carried out at room temperature. Mixing was carried out to remove all lumps from the mixture and to obtain a homogeneous suspension. After 2 hours, the mixture contained no lumps and the solution was a homogeneous suspension. The homogeneity was verified organoleptically under the light. The solution was left in the refrigerator for further analyses.

It was verified that when the time was extended to 3h, the solution was also homogeneous, without lumps, and had the same properties as the solution prepared in 2h.

### Manual method with roller

Alternatively, the method was carried out using a roller. In the initial stage, it is advantageous to wet the BSA powder by gently manually repositioning and kneading the contents of the bag in the hands so that the powder is distributed throughout the volume of water. In the next step, all lumps of BSA powder should be crushed using a roller. As the example uses a 19 cm x 38 cm bag, the acrylic roller used is 14 cm wide (3/4 of the width of the 19 cm bag).

Rolling was performed as follows. The bag was placed on the work surface. A rolling motion was made back and forth across the top surface of the bag pressing it against the bottom surface of the bag lying on the worktop.

For example, it was rolled 10 times forwards and 10 times backwards without pulling the roller away from the bag, through the middle of the bag width, parallel to the longer dimension. The solution is then allowed to move opposite to the direction of movement of the roller in sections near the edges of the length of the bag. The bag was then inverted so that the top surface was on the countertop and the process was repeated. Then the bag was again turned back to its original position and the rolling operation was repeated. Rolling in this manner was performed for 2h.

Of course, rolling can be done in a slightly different way. Rolled 10 times forwards and 10 times backwards, through the centre of the width of the bag, parallel to the longer dimension. The solution is then allowed to move against the direction of movement of the roller in sections near the edges of the length of the bag. The bag was then inverted so that the top surface was on the countertop while rotating it 90° clockwise or anti-clockwise and the process was repeated but directing the roller along the width of the bag (parallel to the shorter dimension). The bag was then turned again to its original position and the operation repeated. Rolling in this manner was performed for 2h.

Of course, rolling can be done in a slightly different way. Rolled 10 times forwards and 10 times backwards, through the centre of the width of the bag, parallel to the longer dimension. The solution is then allowed to move opposite to the direction of the roller's movement in sections near the edges of the bag's length. The bag was then rotated 90° clockwise or anti-clockwise and rolled 10 times forward and 10 times backward, through the centre of the length of the bag, parallel to the shorter dimension. The solution is then allowed to move opposite to the direction of the roll in sections near the edge of the bag width. The bag was then inverted so that the top surface was on the countertop and the operation was repeated, directing the roller along the width of the bag (parallel to the shorter dimension). The bag was then turned back to its original position and the process repeated. Rolling in this manner was performed for 2h.

Each of the above-mentioned rolling methods led to the same result.

In both cases, one movement of the roller in one direction takes about 1-2 s. From the point of view of achieving the objective, it is not so important whether it is 1s, 2s or a time between 1-2s. Depending on the number and distribution of lumps, a longer time, such as 3s, can also be used.

Using a roller that is narrower than the width of the bag allows the solution to flow in sections at the edges of the bag which further allows the solution to be mixed so that a new batch of solution will be rolled out with each roller movement. This allows all lumps of BSA in the solution to be crushed within 2h.

The roller, which is rolled along the bag of mix, is supposed to break up/crush the lumps in the mix. As a result of the lumps being kneaded with the roller, so that the top surface of the bag on the roller side meets the bottom surface of the bag on the tabletop side, the lumps spill inside the bag and are further crushed.

The process was carried out at room temperature. Mixing was carried out to remove all lumps from the mixture and to obtain a homogeneous suspension. The homogeneity of the mixture was evaluated organoleptically under a light, assessing whether the mixture was homogeneous and free of undissolved fragments. After 2 hours, the mixture was free of lumps and the solution was a homogeneous suspension. The solution was left in the refrigerator for further analyses.

It was verified that when the time was extended to 3h, the solution was also homogeneous, without lumps, and had the same properties as the 2h stirred solution. Almost no foam is observed in the solution.

The manual method using either a hand or a roller led to the same solution 1a, with the same organoleptic characteristics and other parameters determined according to the methods described below. This was confirmed in independent experiments, which are not described here. Therefore, for simplicity's sake, the solution obtained manually using either a hand or a roller is called 1a (similarly for 1b and 1c).

### Solution 1b

Description of preparation analogous to the preparation of Solution 1a, whereby: a measured amount of water (1000 ml) was introduced into the bag and then BSA powder (900 g) was poured in so as to obtain a final solution of 60% w/v.

### Solution 1c

Description of preparation analogous to the preparation of Solution 1a, whereby: a measured amount of water (1000 ml) was introduced into the bag and then BSA powder (450 g) was poured in so as to obtain a final solution of 30% w/v.

### Example. 2 Reference examples

### Solution 2

A measured amount of water (100 ml) was poured into a glass laboratory vessel (e.g. https://www.bionovo.pl/p/butelki-laboratorvine-ze-szkla-duran-z-zakretka-o-poi-10-ml-20-I/).

A measured amount of BSA powder (67.5 g) to reach a concentration of 45% w/v was left in the closed laboratory bottle for 10 minutes after weighing. Dissolution of the BSA powder was carried out by gradually adding it to the water bottle. The solution with BSA powder was cycled for 3 days by gently rotating the bottle for 5 min, every 30 min during the 8h operation of each of the 3 days. The process was carried out until a homogeneous mixture was achieved. The homogeneity of the mixture was evaluated organoleptically under a light, assessing whether the mixture was homogeneous and free of undissolved fragments. [https://phb1.com/content/uploads/2023/05/Dissolving-BSA-Into-Solution-White-Paper.pdf.]

### Solution 3

A measured amount of water (100 ml) was poured into a glass laboratory vessel (e.g. https://www.bionovo.pl/p/butelki-laboratoryine-ze-szkla-duran-z-zakretka-o-poj-10-ml-20-I).
The total measured amount of BSA powder (67.5 g) to reach a concentration of 45% w/v was added simultaneously to the water. Shaking of the BSA solution was carried out using a shaker [https://www.e-biosan.pl/Wytrzasarki-kolyski/wytrzasarka-orbitalna-psu-10i.html] at 250 rpm until a homogeneous mixture was achieved. The homogeneity of the mixture was evaluated organoleptically under a light, assessing whether the mixture was homogeneous and free of undissolved fragments. The process was carried out for 3 days.

### Solution 4

A measured amount of water (2000 ml) was poured into a glass laboratory vessel (e.g. https://www.bionovo.pl/k/szklo-i-porcelana/zlewki-szklane/). The total measured amount of BSA powder (1350 g) until a concentration of 45% w/v was reached was gradually added to the water. Using a mixer https://www.silverson.co.uk/en/products/laboratory-mixers/, the dissolution process was carried out at a speed setting of 1500 to 6000 rpm (different settings were tried). The rotor/stator mixer works on the principle of multi-stage mixing/shearing when the materials are drawn in. Centrifugal force then drives the materials towards the periphery of the working head, where they are subjected to milling in the precisely made clearance between the ends of the rotor blades and the inner wall of the stator. Intense hydraulic shearing then takes place as the materials are pushed at high speed through perforations in the stator and into the main body of the mixer. It was not possible to achieve complete dissolution. Therefore, the experiment was discontinued during.

### Solution 5

A measured amount of water (1,600 ml) and BSA powder (1,080 g) to produce a 45% w/v solution was placed in a stirrer [https://www.silverson.co.uk/en/products/flashmix-powder-liquid-mixers/.]
This device used a tank with a pipe drawing liquid from the tank in a 'closed' system, the liquid circulating throughout the system, which was closed except for a small hole to which a funnel was attached. The liquid is drawn from the tank through a pipe into a mixing element (like a blade impeller) whereupon the ejected liquid is forced back into the tank after meeting the powder dispensed through the funnel . It was not possible to achieve complete dissolution. So the experiment was discontinued during.

### Solution 6

A measured amount of water (530 ml) and BSA powder (360 g) to make a 45 w/v solution was placed in a thermomix type mixer [https://www.vorwerk.com/pl/pl/c/strona-glowna/produkty/thermomix/tm6]. The reverse speed was set starting at a setting of 1.5 and was gradually increased to a maximum of 6, without a temperature task. It was not possible to achieve complete dissolution. So the experiment was discontinued during.

### Example 3 Dissolution time

Solution 6 - at the low speed setting above, the powder from above the solution was unable to penetrate into the solution. A hard layer of soaked powder formed at the powder-liquid interface. Increasing the speed above a setting of 6 resulted in a compacted foam, which decried the continued use of this device. In addition, during stirring, the temperature of the solution spontaneously exceeded 40° C, which in the case of protein can lead to denaturation.

Solution 5 - soaked BSA powder at the bottom of the funnel caused a hard layer to form, resulting in clogging of the mixer. As a result, further use of the device was not warranted.

Solution 4 - irrespective of the speed setting, which, if too low (1500 rpm), the powder was unable to pass through the powder-liquid interface, or if too high (6000 rpm), excessive foaming was observed, further use of the unit was not justified.

Solution 3 - after 3 days of dissolution at room temperature, the solution became homogeneous. Foaming of the solution was observed during stirring. Solution 3 was used for further testing as a reference solution.

Solution 2 - after 3 days of gradual dissolution at room temperature, the solution became homogeneous. Foaming of the solution was observed during stirring. It was used for further studies as a reference solution.

Solution 1a, 1b, 1c - after 2 hours of stirring (kneading) as described above at room temperature, the solution became homogeneous. A negligible amount of foam was observed. It was transferred to the refrigerator, until Solutions 2 and 3 were dissolved, in order to start parallel tests.

Conclusions: the use of commercially available mixing systems (Solution 4, 5 and 6) of bovine albumin in water to a high concentration equal to or higher than 30% w/v is not effective. Only in the case of solutions 1a, 1b and 1c prepared according to the inventive method was it observed to obtain a homogeneous suspension, with a negligible amount of foam, in a short time of 2h. In the methods from the state of the art, where it was possible to obtain a homogeneous solution in 3 days (Solution 2 and 3), other disadvantages were observed, already mentioned above and further explained by means of Examples 4, 5, 6.

### Example 4 Microbiological testing

The tests were performed according to ISO 11737-3. Under a BSL-2 class laminar flow cabinet [https://e-alpina.pl/komory-bio-b2], 1 ml of each of the test solutions, i.e. solution 1a obtained according to the invention and reference solutions 2 and 3, was taken. Appropriate dilutions were prepared in sterile PBS (by diluting with PBS buffer 10x and 100x). The prepared dilution series were distributed onto TSA solid media dishes, dispensing 100 µl of diluted solutions per dish. The dishes were placed in an incubator set at 37° C for 24 h. Using the eye, the colonies formed were counted. Samples were analysed in 3 replicates.

Results for a dilution of 100x:
Solution 1a: 24 colonies
Solution 2: 382 colonies
Solution 3: 352 colonies
Conclusions: The lowest microbial load was obtained for Solution 1a, more than 10 times lower than for the samples from the reference solutions, which is probably due to the significantly reduced mixing time.

### Example 5: Determination of monomeric purity of BSA by SDS-PAGE

BSA solutions were prepared from stock solutions 1a, 2 and 3 by diluting to a concentration of 0.045 w/v with water, then mixed with Laemmli Sample Buffer at a ratio of 1:1. The incubation was set to 95 °C and 5 minutes in https://www.eppendorf.com/pl-pl/Sklep-Produkty/Kontrola-temperatury-i-mieszania/Instrumenty/Eppendorf-ThermoMixerC-p-PF-19703 The Mini-Protean Tetra Cell kit was prepared, and the inner and outer chambers were filled with diluted TGS buffer. The purchased TGS buffer was diluted 10x for use, taking in this case 100ml of this buffer and 900ml of water). The wells of the gel were washed with the diluted buffer and 10 µl of solution was dispensed per well. Electrophoresis was carried out for 40 min at 200 V. The gel was removed from the cassette and washed with water, then stained with Bio-Safe Coomassie G-250 for 2 hours. The gel was left to soak in water overnight in the refrigerator. In the morning, the gel was removed from the water and dried with a paper towel, then scanned on an HPM1132 multifunctional laser (mono) printer [https://allegro.pl/oferta/drukarka-laserowa-hp-wielofunkcyjna-mono-hp-m1132-oryginalny-toner-kable-15166591800?utm_feed=aa34192d-eee2-4419-9a9a-de66b9dfae24&utm_content=high-asp&utm_source=google&utm_medium=cpc&utm_campaign=_elktrk_komputery-top_pla_pmax&ev_campaign_id=18869915682&gad_source=18&gclid=CjwKCAjw88yxBhBWEiwA7cm 6pa7ktdOrKwDM0r7EAiEZ5Dll6uWpSNfbJCfUjr7a5zJab-7YR3JsbRoCl3lQAvD_BwE], which was analysed using ImageJ software [https://imagej.net/ij/], measuring stripe intensities to represent the percentage of BSA monomer content. BSA standard was used as a control. Samples were analysed in triplicate.

Results - percentage of monomers:
Solution 1a: 96,9 %
Solution 2: 89.4%
Solution 3: 83.2%

Conclusions: The highest BSA monomer content was obtained for Solution 1a, other oligomeric structures were observed in reference solutions 2 and 3, which are probably the result of prolonged mixing and foaming during dissolution.

### Example 6. UV-VIS Spectrophotometry

Dilutions of solutions 1a, 2 and 3 were prepared to a concentration of 5% w/v albumin in water, a freshly prepared 5% w/v BSA solution was used as a standard (the preparation of such a solution is not problematic and can be done by any known method from the state of the art, e.g. as solutions 2 or 3 but much shorter). 100 µl of the solutions were each applied to a 96-well plate. Water was used as a blank to prepare the BSA solution. Using a spectrophotometer, the absorbance values of each sample were determined in the wavelength range from 230 to 700 nm. The determination was carried out on a Bio-Tek uQuant (equipment as above) using Gen5 software. Samples were analysed in triplicate. The averaged results including the blank are shown in Fig. 1.

Superimposed on Fig. 1 are graphs showing the wavelength dependence of absorbance for the four solutions. It can be seen that the graph for solution 1a (dark grey dashed line) lies closest to the graph for the standard (light grey dotted line). The graphs for solution 2 (light grey solid line) and 3 (black solid line) deviate more significantly from the graph for the standard.

Conclusions: The absorbance values measured for the UV-VIS wavelength range shown in the graph according to Fig. 1 show that Solution 1a is most similar in composition/structure of the compounds present to the spectrum of the standard. This is most likely due to the fact that Solution 1a was obtained by the least destructive means of dissolving BSA in water, which translates into the maintenance of the correct original spatial structure of BSA.

### Example 7: Test of adhesive strength

A two-chamber syringe [https://www.medmix.swiss/en/Products/Surgery/Advanced-biomaterial-mixing-devices/Double-Syringe-Prefilled-Delivery-System] was used to place 4 ml of Solution 1a in the larger chamber and 1 ml of a 10% w/v solution of glutaraldehyde [https://us.vwr.com/store/product/22071133/glutaraldehyde-10-in-aqueous-solution-em-grade]. The adhesive bond strength was then tested in accordance with ASTM F2255 using a testing machine (Tensile Testing Machine CMT-05L - https://trends.directindustry.com/jinan-liangong-testing-technology-co-ltd/project-180963-180394.html) similarly to the publications https://www.ncbi.nlm.nih.gov/pmc/articles/PMC8896806/ or https://www.ncbi.nlm.nih.gov/pmc/articles/PMC3027843/

The test was conducted in accordance with ASTM F2255. The test used frozen pig skin (https://www.fishersci.com/shop/products/porcine-skin-sheet-1-24-sheets/NC1275387), which was cut with a scalpel to 2.5 x 5 cm. The tissue was bonded to the appropriate mounting component using cyanoacrylate adhesive (https://allegro.pl/oferta/klei-kropelka-2ml-14624548186?utm feed=aa34192d-eee2-4419-9a9a-de66b9dfae24&utm source=google&utm medium=cpc&utm campaign= dio przemysl-biuro pla pmax&ev campaign id=17967348209&gad source=1&gclid=CiwKCAiw88vxBhBWEiwA7c m6pa3WI-x57pGpLIWsaLaAwK9BG33Dp O0gmyni-cI8NZ3RXaHi8 XZRoCKR4QAvD BwE). The adhered skin pieces were wrapped in gauze soaked in PBS, placed in a string bag, sealed and placed in an incubator set at 37 °C (https://www.binder-world.com/pl-pl/produkty/hodowla/inkubatory-standardowe/produkt/bd-115) until the start of the study. The skin samples were removed from the freeze, dried with gauze. An even amount of glue was applied to an area 1 cm wide, the glued pieces were joined together. It was left for 5 minutes until full bonding was achieved. The bonded pieces were wrapped in gauze soaked in PBS, placed in a drawstring bag, sealed and placed in an incubator set at 37 °C for 1 h. Samples were removed from the incubator and left at room temperature for 15 min. The glued parts were assembled in a testing machine (https://trends.directindustry.com/jinan-liangong-testing-technology-co-ltd/project-180963-180394.html). The tensile speed was set to 5 mm/min, the maximum force required to break the glued parts was measured. The results are shown in the table below.

| **Measurement number** | **Maximum retained load [N]** |
|---|---|
| **1** | **24.08** |
| **2** | **22.01** |
| **3** | **34.77** |
| **4** | **26.41** |
| **5** | **32.82** |
| **6** | **26.84** |
| **7** | **15.42** |
| **8** | **17.75** |
| **9** | **19.81** |
| **10** | **19.13** |
| **average** | **23.90 (standard deviation 6.05)** |

In a similar manner, the maximum retained load was tested for the adhesive sample made with Solution 2 - a value of 19.08 N was determined for it.

An increase of more than 20 % in the maximum load retention was observed for the adhesive sample according to the invention compared to the adhesive sample obtained by the prior art of method. This demonstrates the better adhesive properties of the adhesive obtained from the bovine albumin solution made by the method according to the invention.

For solutions 1b and 1c, analogous tests were carried out (as example 4, 5, 6 and 7). It was found that solutions with a concentration of 30% w/v and 60% w/v were possible to obtain by the method according to the invention while retaining improved properties with respect to analogous solutions from the state of the art, exhibited the inventive effect analogously.

Analogous studies have shown that, like solution 1a, solutions 1b and 1c are also obtained in 2h to 3h rather than several days, that they are characterised by reduced bacterial counts, a similar graph of the dependence of the absorbance value measured against the UV-VIS wavelength range to the standard, more than 95% monomer content in the SDS-Page test.

### Example 8. Adhesive composition and application

As already mentioned, the adhesive composition according to the invention was obtained as a combination of two solutions: 45% w/v aqueous solution of bovine albumin obtained by the method according to Example 1a and 10% w/v aqueous solution of glutaraldehyde, in a ratio of 4:1, by placing these solutions in a two-chamber syringe providing two chambers with just this volume ratio of fluids (e.g. 4:1 syringe from Medmix https://www.medmix.swiss/Products/Surgery/Advanced-biomaterial-mixing-devices/Double-Syringe-Prefilled-Delivery-System).

The composition in the syringe prepared in this way can be directly applied, if necessary, to glue tissues together during surgery. Squeezed from the syringe, the two components of the composition are mixed immediately before application to the area prepared for bonding. The drying time of the adhesive is 20-30 seconds and full bond strength is achieved within 2 minutes.

## Claims

1. A method of manufacturing an aqueous solution of bovine albumin of 30-60% w/v, where the bovine albumin powder is mixed with the aqueous solution, **characterised in that** the mixing is carried out as follows:
a. an aqueous solution and bovine albumin powder in such quantities as to ensure a concentration of 30-60 % w/v are introduced into a sterile plastic bag,
b. the bag is closed tightly to remove the air inside the bag,
c. optionally, the powder is initially spread manually in the hands with water, so that the powder is distributed throughout the volume of the water solution,
d. manual removal of all lumps of bovine serum albumin powder in aqueous solution is carried out for 2-3h by crushing the lumps between the surfaces of the bag.

2. Method according to claim 1, **characterised in that** the manual removal of all lumps of bovine albumin powder in aqueous solution is carried out with the hands by crushing these lumps with the fingers between the surfaces of the bag.

3. Method according to claim 1, **characterised in that** the manual removal of all lumps of bovine albumin powder in aqueous solution is carried out by crushing the lumps by rolling with a roller.

4. Method according to claims 1 or 2 or 3, **characterised in that** the aqueous solution is water, diluted buffer and/or salt solution.

5. Method according to claims 1 or 2 or 3 or 4, **characterised in that** the resulting aqueous solution of bovine albumin is a 45% w/v solution.

6. An adhesive composition comprising an aqueous solution of bovine albumin prepared by the method according to any of claims 1-5 and an aqueous solution of a di- or polyfunctional aldehyde.

7. Adhesive composition according to claim 6, **characterised in that** the aqueous solution of bovine albumin is an aqueous solution of 45% w/v, the solution of di- or multifunctional aldehyde is an aqueous solution of 10% w/v of glutaraldehyde and the ratio of bovine albumin and glutaraldehyde solution is 4:1.

8. Use of an aqueous solution of bovine albumin prepared by the method according to any of claims 1-6 for the preparation of a tissue adhesive.
